Europäisches Patentamt

European Patent Office

Office européen des brevets

(19)

(11) Publication number: **0 105 579**
**B1**

(12) **EUROPEAN PATENT SPECIFICATION**

(45) Date of publication of patent specification: 07.01.87    (51) Int. Cl.⁴: **A 61 M 1/34**

(21) Application number: 83304513.1

(22) Date of filing: 04.08.83

(54) Adsorber for haemoperfusion.

<table>
<tr><td>(30) Priority: 10.08.82 JP 137914/82</td><td>(73) Proprietor: Japan Medical Supply Co. Ltd.<br>12-17 Kako-Machi,<br>Hiroshima, 733/Japan (JP)</td></tr>
<tr><td>(43) Date of publication of application:<br>18.04.84 Bulletin 84/16</td><td>(72) Inventor: Sugiyama, Masafumi<br>27-4-97-206, Kamesaki, Koyo-Cho<br>Asakita-ku Hiroshima (JP)<br>Inventor: Kawanishi, Hideki<br>Takami Building 503, 3-11, 1-Chome<br>Kokutaiji, Naka-ku Hiroshima (JP)</td></tr>
<tr><td>(45) Publication of the grant of the patent:<br>07.01.87 Bulletin 87/02</td><td></td></tr>
<tr><td>(84) Designated Contracting States:<br>DE FR GB SE</td><td>(74) Representative: Crampton, Keith John Allen<br>et al<br>D YOUNG & CO 10 Staple Inn<br>London WC1V 7RD (GB)</td></tr>
<tr><td>(56) References cited:<br>DE-B-2 705 734<br>GB-A-1 577 714<br>GB-A-1 591 393<br>GB-A-1 603 865</td><td></td></tr>
</table>

Courier Press, Leamington Spa, England.

## Description

This invention relates to adsorbers for haemoperfusion to remove by adsorption poisonous substances contained in blood, and to their production.

In patients suffering from renal failure or liver failure, the ability to excrete poisonous substances, such as creatinine, uric acid, urea and protein-bound substances, from their blood is reduced. This results in an accumulation of such poisonous substances in their blood, which endangers their lives. To prevent this, dialysis or use of adsorbents has previously been used in order to remove the poisonous substances and thereby decontaminate the blood. However, since dialysis involves using a semipermeable membrane, it is rather difficult to effectively remove substances having medium-sized or large molecules and protein-bound substances such as bilirubin.

Another means of decontamination is to bring an adsorbent into direct contact with the blood to adsorb poisonous substances. The poionous substances removed depend on the selection of the adsorbent. With this type of decontamination, the adsorption of poisonous substances contained in blood is carried out by liquid-phase adsorption. In general, it is desirable to use such an adsorbent in the form of powder or fine particles having a large surface area, because substances have a smaller diffusion velocity in the liquid phase than in the gaseous phase, and the adsorption velocity is consequently dependent on the surface area of the adsorbent. However, adsorbents in the form of fine particles having a large surface area cannot be used safely, because they would flow into the human body. Thus, in order to effectively decontaminated blood by the use of an adsorbent in the form of powder or fine particles, it becomes essential to fix the adsorbent by some effective means. Several methods have been proposed for mixing a high-molecular material and an adsorbent and fixing them into a spherical or membranous form, as described in Artificial Organs, Vol. 4,302 (1980) and Vol. 6,151 (1982) and Japanese Patent Publication No. 32636/1978. These methods are inadequate because the ratio of the high-molecular-weight material to the adsorbent has to be great enough to adequately maintain such a fixed state, with the result that the high-molecular material layer on the surface of the adsorbent will become thicker and the adsorptivity will be greatly reduced.

A method of using a particulate adsorbent coated with a thin layer of a high-molecular material has been proposed in Japanese Patent Publication Nos. 3944/1980 and 27090/1981. This method is still inadequate because poisonous substances are transformed so slowly into the absorbent that a satisfactory adsorption velocity can hardly be expected.

GB—1591393 describes a membrane unit for substantially simultaneously removing from blood toxic metabolites and metabolites normally present in urine, which unit comprises a passage for blood and a permselective membrane which provides access therethrough to a compartment in which a filtrate can be absorbed and/or transported and, if desired, in which a rinsing solution can be transported, the passage lying between and being defined by the permselective membrane and an adsorbent material or a member contacting an adsorbent material and providing access through the wall of the member to the adsorbent material, which adsorbent material, whether defining the passage or contacting the member is not positioned in the said compartment. The adsorbent material may be enveloped by or embedded in a porous membrane. The permselective membrane of this unit may be applied to a reinforcing and supporting carrier which may be *inter alia* a mesh net of a synthetic material.

The present invention provides an adsorber for haemoperfusion comprising a porous membrane containing an adsorbent in the form of powder of fine particles characterised in that the membrane has a thickness of 10 to 400 μm and a support having a net structure is embedded in the membrane.

The support is used to increase the strength of the whole adsorber, so that the adsorber can be assured of a strength that is sufficient in practice, even when the high-molecular-weight material for fixing the adsorbent is used in decreased amount. Thus, a high adsorption velocity can be obtained because the high-molecular-weight material is used in a small amount.

The adsorbents suitable for use in an adsorber in the present invention include activated carbon, porous resin, porous alumina, porous silica, porous glass and ion-exchange resins. These adsorbents should be selected depending on the substances to be removed from the blood. The adsorbent must be in the form of powder or fine particles, whereby a high adsorption velocity can be obtained. The suitable average particle diameter of the adsorbent is 10 to 100 μm.

The support of this invention is used for fixing the adsorbent. It has a net structure which serves to keep the blood circulating under good conditions. The most desirable support has a net structure having portions at its intersections of greater thickness than the portions connecting the intersections, i.e. thick particulate portions and thin linear portions. Normally, the adsorber is used in multiple layers. The superposed adsorber layers, each of which is provided with a support having a structure as described above, will retain a suitable space between one another without clinging to each other, so that the entire adsorber will admit the blood in a uniform flow, whereby a high adsorption velocity can be obtained and the pressure loss can be minimized. Moreover, the fact that a large surface area is obtained is advantageous in respect of adsorption velocity.

The invention will now be described with reference to the accompanying drawings, which are illustrative. In the drawings:

Figure 1 is a diagram showing an enlarged front view of an embodiment of a support used for an adsorber of this invention;

Figure 2 is a diagram showing a cross-section across A—A in Figure 1;

Figure 3 is a diagram showing the disassembled components of a haemoperfusion device having a built-in adsorber in accordance with this invention, prior to assembly in a housing vessel (not shown);

Figure 4 is a cross-section of the assembly of the components of Figure 3.

When using a device as illustrated, the adsorbent is prevented from intruding into the body and the compatability of the adsorber with blood is excellent.

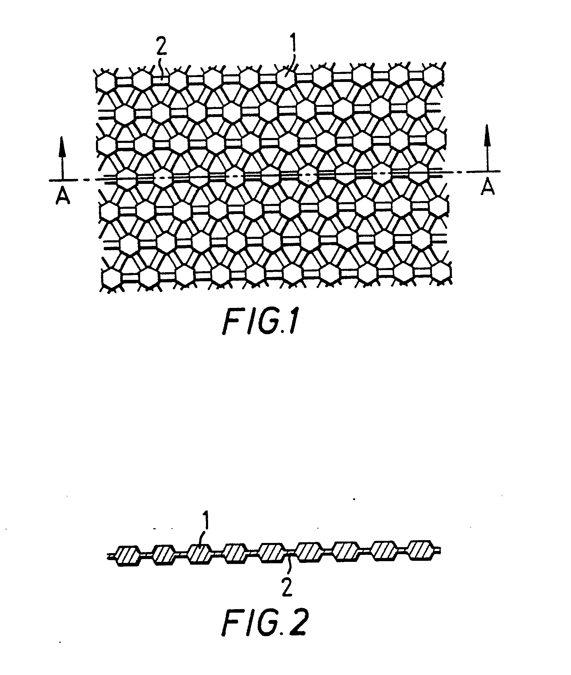

In Figures 1 and 2, 1 is a thick particulate portion and 2 is a thin linear portion.

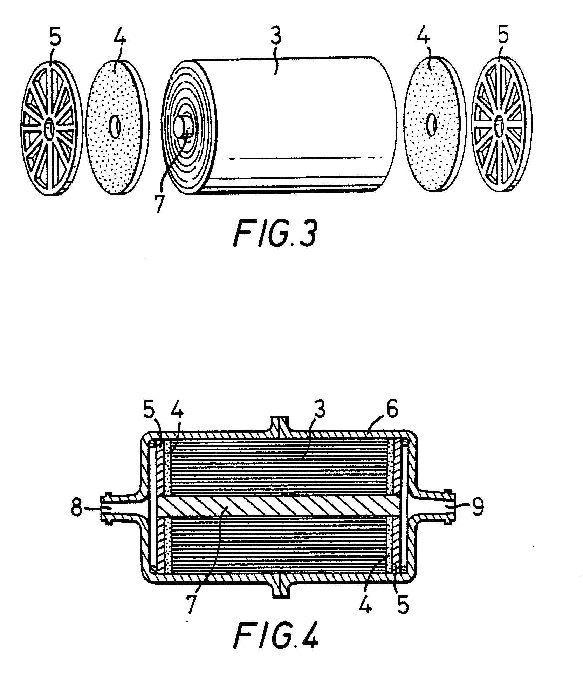

In the Figures 3 and 4, rolled adsorber 3 contains an adsorbent in the form of powder or fine particles fixed on a mesh support. An elastomer 4 is provided on both ends of the adsorber, and end support 5 is provided on both sides of the elastomer to hold the adsorber and the elastomer. Items 3 to 5 are housed in a vessel 6 and core 7 is central.

Preferably, the thick portions in Figures 1 and 2 have a thickness ranging from 40 to 500 μm and the thin portions from 3 to 30 μm. The net structure should have a void content ranging from 30 to 80%. If the void content is smaller than 30%, the pressure loss will increase, while if it is greater than 80%, the amount of the adsorbent to be fixed will decrease. The diameter of the thick portions is preferably in the range from 50 to 1,000 μm, and the width of the thin portions from 5 to 500 μm, although these dimensions need not be particularly specified. The thick portions should be present in a density of 10 to 200/cm².

The above-mentioned support can be prepared by known methods. For instance, the method set forth in Japanese Patent Laid-Open No. 38977/1979 is convenient for the present purpose. The support thus prepared is scattered with polygonal or circular thick portions, from which thin portions extend radially and connect with thick portions (see Figure 1).

The materials for the support are, for example, high-molecular-weight substances, such as polyethylene, polypropylene, nylon, polyester and cellulose, metals and ceramics; above all, high-molecular-weight substances are advantageous in respect of their handling characteristics and easy processability. Moreover, it is desirable for the support to have a tensile strength of 0.2 kg/cm or greater in practical use.

The adsorber of the invention can be prepared by the known method of producing porous membranes by applying a polymer solution, in which the adsorbent is dispersed, to the support by flowcasting, dipping, coating or spraying, and then bringing the dispersion into contact with a non-solvent (i.e. a liquid in which the dispersion is substantially insoluble) for solidifying the polymer and extracting the solvent. The porous membrane containing the adsorbent is then fixed on the support. It is also possible to prepare the adsorber by applying to the support a molten polymer containing the adsorbent and extractable additives dispersed in it, solidifying the polymer, and subsequently extracting the additives, thereby providing a porous structure.

The polymers capable of forming a porous membrane include polyurethane, polysulphone, polystyrene, polyamide, polyethylene, polypropylene, polyvinyl alcohol, polyhydroxyethyl methacrylate and cellulose.

Though the content of the adsorbent in the adsorber is not particularly specified, it should normally be in the range 30 to 70 wt%, in view of the fact that its adsorption capacity will decrease when its content is too low and that its fine particles will occur increasingly when its content is too high.

In this invention, the porous membrane fixed on the support is required to have a thickness ranging from 10 to 400 μm. If the membrane thickness is greater than 400 μm, the adsorbent will not be dispersed uniformly inside the porous membrane, so that its density distribution will become uneven in some portions of the adsorber. As a result, the diffusion of the adsorbed substances inside will become slower, and both the adsorption velocity and the adsorption capacity will decrease. On the other hand, if the membrane thickness is smaller than 10 μm, the membrane strength will decrease, and it is possible that the membrane will be injured while in use. Such damage to the membrane would allow the adsorbent to be mixed with the blood and flow into the body, and could cause blood platelets to adhere to the surface of the adsorber. The term "membrane thickness", as used herein, means the thickness of the membrane measured in the spaces between the elements of the net structure.

Normally, the adsorber of this invention is used by rolling or superposing it into multiple layers and housing it in a vessel having an inlet and an outlet for the blood. The adsorber may be contacted with the whole blood or with plasma or serum alone.

The elastomer is provided to protect the end portions of the adsorber, and has a porous structure so as not to interfere with the passage of blood. More precisely, it can be an open-cell foam consisting of a high-molecular-weight material such as polyurethane or silicone. Since the device is provided with the elastomer, the fine adsorbent particles can be prevented to a large extent from leaving at the end portions of the adsorber, and they can therefore serve effectively for haemoperfusion. The elastomer can be, for example, circular, oval square or oblong in shape, corresponding to the shape of the end portion of the adsorber.

In the embodiment illustrated, the elastomer is provided with an end support on both sides. The end support should consist of a rigid material and have a structure for holding the adsorber and the elastomer entirely and creating space for passage of blood, such as a porous board or a structure as shown in Figure 1. The end support enables the elastomer to uniformly adhere to the entire end portion of the adsorber, so that the elastomer will be effective across its entire surface. The support is preferably made of a plastics material such as polyethylene, polypropylene or polycarbonate. Alternatively, metals or ceramics may be used.

The haemoperfusion device is thus built by assembling the above-mentioned absorber, elastomer and end support, and housing them in the vessel having an inlet and an outlet for the blood. The housing vessel 6 is provided with a blood inlet 8 and outlet 9. The vessel can be made of various plastics materials such as polystyrene, polypropylene or polycarbonate, metals glass or ceramics, and may be composed of two or more portions as necessary.

The haemoperfusion device should be sterilized prior to use, by means of steam or γ-ray irradiation.

The following Examples illustrate the preparation and use of an adsorber in accordance with the present invention.

Example 1

10 g of powdery activated carbon (particle diameter of 40 µm or less) was mixed with 50 ml of a polyurethane solution (a solution of 10 w/v% polyurethane in tetrahydrofuran) to give a suspension, which was allowed to flow on both sides of a polyethylene support (thick portions 140 to 160 µm in thickness, thin portions 8 to 12 µm in thickness, thick portions 90 to 100/cm² in number, void content 40 to 45%, and tensile strength 0.6 to 0.8 kg/cm wid.) which has a net structure as shown in Figures 1 and 2, whereby a 200 µm thick membrane was prepared. The membrane was then dipped in water, so as to extract tetrahydrofuran that it contained. It was freed of the solvent completely by dipping in hot water at 80°C to give an adsorber having a porous membrane 200 µm thick. The adsorber thus obtained had an activated carbon content of 65%, with a lot of fine holes, 1 to 6 µm in diameter, on the polyurethane layer.

For comparison, an adsorber having a porous membrane 500 µm thick was prepared using a polypropylene mesh support by the same method as described above.

In the present experiment, mongrel grown-up dogs were caused to suffer from jaundice with their common bile duct ligated, and after three to four weeks their serum was collected as a test solution for the measurement of the adsorptivity of the two different adsorbers prepared as above, powdery activated carbon and granular activated carbon. In testing, 0.25 g of each adsorber or adsorbent was added to 50 ml each of the test solution, and the mixture was stirred at 37°C and 120 cpm or 120 minutes to measure the total bilirubin concentrations before and after the adsorption test. The results are shown in Table 1.

TABLE 1

| | Bilirubin concentration (mg/d) | | |
|---|---|---|---|
| | before adsorption | after adsorption | Adsorption rate % |
| Adsorber (200 µm thick) | 10.5 | 4.7 | 55.2 |
| Adsorber (500 µm thick) | 10.5 | 8.0 | 23.5 |
| Powdery activated carbon | 10.5 | 3.3 | 68.6 |
| Granular activated carbon | 10.5 | 9.5 | 9.5 |

As shown in Table 1, the 200 µm thick adsorber in this invention exhibited an adsorption rate close to that of powdery activated carbon. However, the 500 µm thick porous membrane afforded a rate of adsorption that was only about a half of the value obtained with the membrane 200 µm thick, and it is evident that the adsorbtivity is affected greatly by the thickness of porous membrane.

Example 2

100 g powdery activated carbon (particle diameter of 10 to 40 µm) was mixed with 500 ml of a polyurethane solution (a solution of 10 w/v% polyurethane in tetrahydrofuran) to give a suspension, which was allowed to flow on both sides of a polyethylene support (thick portions 140 to 160 µm in thickness, thin portions 8 to 12 µm in thickness, thick portions 90 to 100/cm² in number, void content 40 to 45%, and tensile strength 0.6 to 0.8 kg/cm wid.) which has a net structure as shown in Figures 1 and 2, whereby a membrane 200 µm thick was prepared. The membrane was then dipped in water so as to extract tetrahydrofuran that it contained. It was freed of the solvent completely by dipping in hot water at 80°C to give an adsorber. The adsorber thus obtained had an activated carbon content of 65%, with a lot of fine holes, 1 to 6 µm in diameter, on the polyurethane layer. About 40 m of this adsorber was rolled, and a haemoperfusion column as shown in Figures 3 and 4 was prepared with it. The adsorber housed in the column contained about 80 g of activated carbon.

The whole blood of dogs was passed through this haemoperfusion column at a flow rate of 50 ml/min, and its pressure was observed at the inlet and the outlet of the column. The pressure difference, i.e. pressure loss, in this case was found to be 15 mmHg. Such a small pressure loss will not cause haemolysis. In contrast, the pressure loss observed in the case of a commercially available haemoperfusion column packed with granular activated carbon reached as high as 100 mmHg. However, there was very little difference between the two cases in the amount of carbon dust occurring. Thus, the adsorber in this invention can be used safely for haemoperfusion.

## Claims

1. An adsorber for haemoperfusion comprising a porous membrane containing an adsorbent in the form of powder or fine particles characterised in that the membrane has a thickness of 10 to 400 µm and a support having a net structure (1, 2) is embedded in the membrane.

2. An adsorber as claimed in claim 1, in which the net structure has portions at its intersections of greater thickness than the portions connecting the intersections.

3. An adsorber as claimed in either of claims 1 and 2, in which the adsorbent has an average particle diameter ranging from 10 to 100 µm.

4. An adsorber as claimed in any one of claims 1 to 3, in which the adsorbent is activated carbon or a porous organic resin.

5. An adsorber as claimed in any one of claims 1 to 3, in which the adsorbent is porous alumina, porous silica or porous glass.

6. An adsorber as claimed in any one of claims 1 to 5, in which the porous membrane comprises polyurethane.

7. An adsorber as claimed in any one of claims 1 to 6, in which the porous membrane contains the adsorbent in the range from 30 to 70 wt%.

8. A method of preparing an adsorber as claimed in claim 1, comprising applying a polymer solution in which an adsorbent in the form of powder of fine particles is dispersed to a support, having a net structure (1, 2), and subsequently contacting the thus treated support with a non-solvent for polymers.

9. A method as claimed in claim 8, in which the non-solvent is water.

## Patentansprüche

1. Adsorber für die Hämoperfusion mit einer porösen Membran, die ein Adsorbens in der Form von Pulver oder feiner Teilchen enthält, dadurch gekennzeichnet, daß die Membran eine Dicke von 10 bis 400 µm besitzt und ein Träger mit einer Netzstruktur (1, 2) in die Membran eingebettet ist.

2. Adsorber nach Anspruch 1, bei dem die Netzstruktur an ihren Schnittpunkten Bereiche größerer Dicke als die die Schnittpunkte verbindenden Bereiche hat.

3. Adsorber nach einem der Ansprüche 1 und 2, bei dem das Adsorbens einen mittleren Teilchendurchmesser im Bereich von 10 bis 100 µm hat.

4. Adsorber nach einem der Ansprüche 1 bis 3, bei dem das Adsorbens Aktivkohle oder ein poröses organisches Harz ist.

5. Adsorber nach einem der Ansprüche 1 bis 3, bei dem das Adsorbens poröses Aluminiumoxid, poröse Kieselsäure oder poröses Glas ist.

6. Adsorber nach einem der Ansprüche 1 bis 5, bei dem die poröse Membran Polyurethan umfaßt.

7. Adsorber nach einem der Ansprüche 1 bis 6, bei dem die poröse Membran das Adsorbens im Bereich von 30 bis 70 Gew.-% enthält.

8. Verfahren zur Herstellung eines Adsorbers nach Anspruch 1, wobei man eine Polymerlösung, in welcher ein Adsorbens in der Form von Pulver oder feiner Teilchen dispergiert ist, auf einem Träger mit einer Netzstruktur (1, 2) aufbringt und anschließend den so behandelten Träger in Berührung mit einem Nichtlösungsmittel für Polymere bringt.

9. Verfahren nach Anspruch 8, bei dem das Nichtlösungsmittel Wasser ist.

## Revendications

1. Adsorbeur pour hémoperfusion, comprenant une membrane poreuse contenant un adsorbant sous la forme d'une poudre ou de fines particules, caractérisé en ce que la membrane a une épaisseur de 10 à 400 µm, et qu'un support ayant une structure réticulée (1, 2) est noyé dans la membrane.

2. Adsorbeur selon la revendication 1, dans lequel la structure réticulée possède des parties, à ses intersections, ayant une épaisseur supérieure à celle des parties qui relient les intersections.

3. Adsorbeur selon l'une quelconque des revendications 1 et 2, dans lequel l'adsorbant a un diamètre moyen de particules compris entre 10 et 100 µm.

4. Adsorbeur selon l'une quelconque des revendications 1 à 3, dans lequel l'adsorbant est du charbon activé ou une résine organique poreuse.

5. Adsorbeur selon l'une quelconque des revendications 1 à 3, dans lequel l'adsorbant est de l'alumine poreuse, de la silice poreuse ou du verre poreux.

6. Adsorbeur selon l'une quelconque des revendications 1 à 5, dans lequel la membrane poreuse comprend du polyuréthanne.

7. Adsorbeur selon l'une quelconque des revendications 1 à 6, dans lequel la membrane poreuse contient l'adsorbant à raison de 30 à 70% en poids.

8. Procédé pour préparer un adsorbeur selon la revendication 1, consistant à appliquer une solution de polymères, dans laquelle est dispersé un adsorbant sous la forme d'une poudre ou de fines particules, sur un support ayant une structure réticulée, (1, 2), puis à mettre en contact le support ainsi traité avec une substance non-solvante vis-à-vis des polymères.

9. Procédé selon la revendication 8, dans lequel la substance non-solvante est l'eau.

FIG.1

FIG.2

FIG.3

FIG.4